# EUROPÄISCHE PATENTSCHRIFT

(11) **EP 4 115 842 B1**
(45) Veröffentlichungstag und Bekanntmachung des Hinweises auf die Patenterteilung: **19.03.2025**
(21) Anmeldenummer: 22181248.0
(22) Anmeldetag: 27.06.2022
(51) Int. Cl.: A61B 90/30, A61B 90/00, A61B 17/00

(54) **BELEUCHTUNGSANORDNUNG**
ILLUMINATION ASSEMBLY
SYSTÈME D'ÉCLAIRAGE

(30) Priorität: 09.07.2021 DE 102021117734
(43) Veröffentlichungstag der Anmeldung: 11.01.2023
(73) Patentinhaber: Karl Leibinger Asset Management GmbH & Co. KG, 78570 Mühlheim an der Donau (DE)
(72) Erfinder: SCHULZ, Harald, 78532 Tuttlingen (DE); ZEHNDER, Sven, 78733 Aichhalden (DE)
(74) Vertreter: Behr, Wolfgang

(56) Entgegenhaltungen:
- WO-A2-2009/002467
- US-A1- 2015 078 615
- US-A1- 2016 007 839

## Beschreibung

Die vorliegende Erfindung betrifft eine Beleuchtungsanordnung, welche mindestens eine Lichtquelle umfasst, mit mindestens einer Kamera und einer Steuerung.

Es ist bekannt, dass bei der Beleuchtung eines Bereichs die Farbe und/oder Farbtemperatur der Beleuchtung entscheidenden Einfluss auf die Sichtbarkeit von in dem Bereich befindlichen unterschiedlichen Strukturen hat.

Im Operationsumfeld bzw. medizinischen Umfeld kann aktuell insbesondere bei Operationsleuchten die Farbe und/oder Farbtemperatur eingestellt werden. So können je nach beleuchtetem Gewebe Unterschiede und Konturen im Gewebe bei unterschiedlichen Farben und/oder Farbtemperaturen unterschiedlich gut sichtbar gemacht werden. Beispielsweise kann der Unterscheid zwischen tumorbefallenem Gewebe und gesundem Gewebe bei einer ersten Farbtemperatur, der Unterschied zwischen Muskelgewebe und Nervengewebe aber bei einer zweiten Farbtemperatur besser sichtbar sein. Hierzu können manuell verschiedene Farben und/oder Farbtemperaturen eingestellt werden, wobei die Wirkung der jeweiligen Farbe und/oder Farbtemperatur durch eine Bedienperson bewertet und anschließend der Operationsbereich mit einer gewünschten Farbe und/oder Farbtemperatur beleuchtet wird. Diese stellt notwendigerweise immer einen Kompromiss dar. Weiterhin kommt im Operationsumfeld bzw. medizinischen Umfeld häufig eine Kamera zum Einsatz, welche ein Bild des Operationsbereiches aufnimmt. Somit kann der Operationsbereich beispielsweise vergrößert dargestellt werden und/oder der Operationsbereich einer Person zugänglich gemacht werden, die keinen direkten Einblick in den Bereich hat. Letzteres ist insbesondere bei einer Endoskopie-Kamera der Fall.

Der soeben hergestellte Bezug zu einem Operationsumfeld ist lediglich beispielhafter Natur und dient der Veranschaulichung der Problemstellung. Die Möglichkeit einer Änderung der Farbe und/oder Farbtemperatur einer Beleuchtung und damit verbundenen Verbesserung der Sichtbarkeit von Strukturen kann ebenso in anderen technischen Gebieten zum Einsatz kommen. Bevorzugt kommt diese dann zum Einsatz, wenn die gute Sichtbarkeit von unterschiedlichen Strukturen in einem beleuchteten Bereich besonders hohe Priorität hat.

Allerdings bringt die eingangs erwähnte Art der Farb- und/oder Farbtemperatureinstellung Nachteile mit sich. Zunächst ist es erforderlich, dass eine Bedienperson das Ändern der Farbe und/oder Farbtemperatur der Beleuchtung manuell durchführt, was aufwändig ist. Weiterhin kann beispielsweise auf einem Kamerabild, welches während der Beleuchtung mit einer spezifischen Farbe und/oder Farbtemperatur aufgenommen wurde, lediglich die durch die spezifische Farbe und/oder Farbtemperatur erzielte vorteilhafte Sichtbarkeit der Strukturunterschiede realisiert werden, während Bereiche der Struktur, die bei einer anderen Farbe und/oder Farbtemperatur gut sichtbar wären, diesbezüglich unvorteilhaft beleuchtet werden.

Aus Druckschrift WO 2009/002467 A2 ist ein endoskopisches Visualisierungssystem bekannt, das ein selektiv reflektierendes Element verwendet, das eine Rückwärts-Betrachtung und Vorwärtsbetrachtung mit einem Endoskop mit einer einzelnen Kamera ermöglicht. Das endoskopische Visualisierungssystem kann von einem Vorwärts-Betrachtungsmodus in einen Rückwärts-Betrachtungsmodus oder in einen Modus umschaltbar sein, der die gleichzeitige Betrachtung von Vorwärts- und Rückwärts-Bildern ermöglicht. Dabei ist angegeben, dass die gescannten Vorwärts- und Rückwärtsansichten einer geeigneten Bildverarbeitung unterzogen werden, um eine integrierte Ausgabe zu erhalten. Die Vorwärts- und Rückwärtsbilder können auf separaten Monitoren angezeigt werden oder in eine einzige Anzeige auf einem Monitor integriert werden. Ein solcher Schritt kann die Synchronisierung des angezeigten Bildes mit der Position des Reflektors sein. Das vordere Bild kann auf ein vom hinteren Bild getrenntes Bild projiziert werden. Es können verschiedene Wellenlängen des Lichts verwendet werden, z. B. für die Diagnose von vermuteten Läsionen. Die Bilder mit den verschiedenen Wellenlängen können separat angezeigt oder in ein einziges Bild integriert werden, z. B. unter Verwendung von Falschfarben, um die Ergebnisse der Gewebeanalyse auf dem Display anzuzeigen.

Druckschrift US 2015/078615 A1 zeigt ein weiteres endoskopisches Verfahren. Aufgabe der vorliegenden Erfindung ist es daher, eine Beleuchtungsanordnung mit verbesserten Eigenschaften bereitzustellen.

Diese Aufgabe wird durch eine Beleuchtungsanordnung gemäß Anspruch 1 gelöst. Bevorzugte Ausgestaltungen der vorliegenden Erfindung sind Gegenstand der Unteransprüche.

Die vorliegende Erfindung umfasst eine Beleuchtungsanordnung, welche mindestens eine Lichtquelle umfasst, mit mindestens einer Kamera und einer Steuerung, wobei die Farbe und/oder Farbtemperatur der Lichtquelle veränderbar ist und die Kamera Bilder eines durch die Lichtquelle beleuchteten Bereichs aufnimmt. Die Steuerung steuert die Beleuchtungsanordnung so an, dass die Lichtquelle den beleuchteten Bereich nacheinander mit Licht unterschiedlicher Farbe und/oder Farbtemperatur beleuchtet, wobei die Steuerung mindestens ein erstes, bei einer ersten Farbe und/oder Farbtemperatur der Beleuchtung aufgenommenes Bild separat auswertet und/oder ausgibt. Somit kann die optische Wirkung der ersten Farbe und/oder Farbtemperatur der Beleuchtung anhand des ersten Bildes beurteilt werden und/oder genutzt werden, ohne die Farbe und/oder Farbtemperatur der Beleuchtungsanordnung dauerhaft auf die erste Farbe und/oder Farbtemperatur einzustellen.

Die vorliegende Erfindung umfasst wie oben beschrieben daher zunächst eine erste Variante, bei welcher die Lichtquelle Weißlicht ausstrahlt, dessen Farbtemperatur veränderbar ist. In einer zweiten Variante strahlt die Lichtquelle dagegen farbiges Licht aus, wobei die Farbe des farbigen Lichts veränderbar ist. Wird daher im folgenden auf die Farbtemperatur Bezug genommen, handelt es sich um die Farbtemperatur des gemäß der ersten Variante ausgestrahlten Weißlichts.

Die Kamera nimmt in einer bevorzugten Ausgestaltung Farbbilder des beleuchteten Bereichs auf.

Gemäß der vorliegenden Erfindung steuert die Steuerung die Lichtquelle in mindestens einem Betriebsmodus so an, dass die Veränderung der Farbe und/oder Farbtemperatur so schnell erfolgt, dass dies für das menschliche Auge bei unmittelbarer Betrachtung des beleuchteten Bereichs nicht wahrnehmbar ist. Somit kann beispielsweise eine solche Veränderung auch während einem laufenden Operationsbetrieb erfolgen, ohne dass ein Operateur bei unmittelbarer Betrachtung des beleuchteten Bereichs durch diese Veränderung der Lichtverhältnisse gestört werden würde.

Bevorzugt kann der soeben erwähnte Betriebsmodus mittels einer manuell bedienbaren Eingabevorrichtung eingeschaltet und/oder ausgeschaltet werden.

In einer möglichen Ausgestaltung der vorliegenden Erfindung ist eine für das menschliche Auge wahrnehmbare Hauptbeleuchtungs-Farbe und/oder Farbtemperatur, mit der die Lichtquelle den Bereich beleuchtet, manuell einstellbar.

Bevorzugt ist die Hauptbeleuchtungs-Farbe und/oder Farbtemperatur mittels einer manuell bedienbaren Eingabevorrichtung einstellbar.

In einer möglichen Ausgestaltung der vorliegenden Erfindung ist die erste Farbe und/oder Farbtemperatur der Beleuchtung, bei welcher ein Bild aufgenommen wird, das separat ausgewertet und/oder ausgegeben wird, einstellbar.

Bevorzugt ist die erste Farbe und/oder Farbtemperatur der Beleuchtung mittels einer manuell bedienbaren Eingabevorrichtung einstellbar.

In einer möglichen Ausgestaltung der vorliegenden Erfindung wird die Lichtquelle in einem Betriebsmodus so angesteuert, dass die Lichtquelle den Bereich mit einer Hauptbeleuchtungs-Farbe und/oder Farbtemperatur beleuchtet und von dieser Hauptbeleuchtungs-Farbe und/oder Farbtemperatur für einen Zeitabschnitt zur ersten Farbe und/oder Farbtemperatur wechselt, wobei während dieses Zeitabschnitts mindestens ein Bild aufgenommen wird, welches separat ausgewertet und/oder ausgegeben wird.

In einer möglichen Ausgestaltung der vorliegenden Erfindung ist der Zeitabschnitt bevorzugt so kurz, dass sich bei unmittelbarer Betrachtung des beleuchteten Bereichs eine wahrgenommene Farbe und/oder Farbtemperatur durch den Wechsel nicht ändert.

In einer möglichen Ausgestaltung der vorliegenden Erfindung ist der Zeitabschnitt weniger als 200 ms, bevorzugt weniger als 50 ms lang.

In einer möglichen Ausgestaltung der vorliegenden Erfindung handelt es sich bei der Lichtquelle um eine LED-Lichtquelle, wobei die LEDs der LED-Lichtquelle mittels der Steuerung gepulst betrieben werden.

In einer möglichen Ausgestaltung der vorliegenden Erfindung steuert die Steuerung die Beleuchtungsanordnung in mindestens einem Betriebsmodus so an, dass sich während der Aufnahme des Bildes die Farbe und/oder Farbtemperatur der Pulse nicht ändert.

Dies ist allerdings nicht zwingend notwendig. Entscheidend ist vielmehr, dass sich die gemittelte Farbe und/oder Farbtemperatur während der Belichtungszeit der separat ausgewerteten und/oder ausgegebenen Aufnahme gegenüber einem vorangegangene und/oder nachfolgendem Zeitraum unterscheidet.

In einer möglichen Ausgestaltung der vorliegenden Erfindung steuert die Steuerung die Beleuchtungsanordnung in mindestens einem Betriebsmodus so an, dass sich während der Aufnahme eines Bildes erfolgende Pulse im Mittel eine gewünschte Farbe und/oder Farbtemperatur ergeben.

In einer möglichen Ausgestaltung der vorliegenden Erfindung steuert die Steuerung die Beleuchtungsanordnung in mindestens einem Betriebsmodus so an, dass die Beleuchtung nacheinander mit einer Mehrzahl von unterschiedlichen Farben und/oder Farbtemperaturen erfolgt, wobei die Steuerung mehrere, bei jeweils unterschiedlichen Farben und/oder Farbtemperaturen der Beleuchtung aufgenommene Bilder separat auswertet und/oder ausgibt.

Bevorzugt erfolgt die Beleuchtung mit einer aus der Mehrzahl von unterschiedlichen Farben und/oder Farbtemperaturen jeweils in einem Zeitabschnitt, der weniger als 200 ms, bevorzugt weniger als 50 ms lang ist.

Bevorzugt kehrt die Steuerung zu einer Hauptbeleuchtungsfarbe und/oder -farbtemperatur zurück, bevor wieder mit einer anderen Farbe und/oder Farbtemperatur beleuchtet wird.

In einer möglichen Ausgestaltung der vorliegenden Erfindung steuert die Steuerung die Beleuchtungsanordnung in mindestens einem Betriebsmodus so an, dass jeweils von einer Hauptbeleuchtungs-Farbe und/oder Farbtemperatur zu einer der unterschiedlichen Farben und/oder Farbtemperaturen und zurück gewechselt wird.

In einer möglichen Ausgestaltung der vorliegenden Erfindung wird in einem Betriebsmodus auf einer Anzeige der Beleuchtungsanordnung ein Bild dargestellt, welches den beleuchten Bereich bei einer anderen Farbe und/oder Farbtemperatur der Beleuchtung zeigt als jene Farbe und/oder Farbtemperatur der Lichtquelle, die während der Darstellung des Bildes bei unmittelbarer Betrachtung des beleuchteten Bereichs wahrnehmbar ist.

Hierdurch hat eine Bedienperson, beispielsweise ein Operateur, die Möglichkeit, den beleuchteten Bereich auf einer Anzeige mit einer anderen Farbe und/oder Farbtemperatur zu betrachten als jener, welche er bei betrachten des beleuchteten Bereichs unmittelbar wahrnimmt. Hierdurch können auf der Anzeige Strukturen, welche bei der unmittelbaren Betrachtung aufgrund der ungünstigen Farbe und/oder Farbtemperatur nicht oder nur schlecht wahrnehmbar sind, auf der Anzeige überprüft werden.

In einer möglichen Ausgestaltung der vorliegenden Erfindung ist die andere Farbe und/oder Farbtemperatur der Beleuchtung, bei welcher das auf der Anzeige dargestellte Bild aufgenommen wird, einstellbar.

In einer möglichen Ausgestaltung der vorliegenden Erfindung werden in einem Betriebsmodus auf einer Anzeige der Beleuchtungsanordnung mehrere Bilder dargestellt, welche den beleuchten Bereich bei jeweils unterschiedlicher Farbe und/oder Farbtemperatur der Beleuchtung zeigen.

Beispielsweise kann ein Bild mit anderer Farbe und/oder Farbtemperatur als der Haupt-Farbe und/oder Farbtemperatur als Bild-im-Bild angezeigt werden.

In einer möglichen Ausgestaltung der vorliegenden Erfindung wird in einem Betriebsmodus auf der Anzeige mindestens ein Bild dargestellt, welches den beleuchteten Bereich bei einer sich in seiner Farbe und/oder Farbtemperatur ändernden Beleuchtung zeigt.

In einer möglichen Ausgestaltung der vorliegenden Erfindung wird die Beleuchtungsanordnung so angesteuert, dass bei einer Änderung einer Farbe und/oder einer Farbtemperatur der Beleuchtung eines auf der Anzeige dargestellten Bildes sich eine bei unmittelbarer Betrachtung des beleuchteten Bereichs wahrgenommene Farbe und/oder Farbtemperatur der Beleuchtung nicht ändert und/oder mindestens ein anderes auf der Anzeige dargestelltes Bild den Bereich mit einer Beleuchtung wiedergibt, deren Farbe und/oder Farbtemperatur sich nicht ändert.

In einer möglichen Ausgestaltung der vorliegenden Erfindung ist eine gewünschte Hauptbeleuchtungs-Farbe und/oder Farbtemperatur, mit der die Lichtquelle den Bereich in einem Normalbetrieb beleuchtet, einstellbar, insbesondere anhand von auf einem Monitor darstellbaren Bildern mit jeweils unterschiedlicher Farbe und/oder Farbtemperatur der Beleuchtung und/oder anhand eines auf der Anzeige dargestellten Bildes mit einer sich ändernden Farbe und/oder Farbtemperatur der Beleuchtung durch eine Bedienperson einstellbar.

In einer möglichen Ausgestaltung der vorliegenden Erfindung ist eine erste Farbe und/oder Farbtemperatur, bei welcher ein Bild aufgenommen wird, das separat ausgewertet und/oder ausgegeben wird, einstellbar, insbesondere anhand eines auf der Anzeige dargestellten Bildes mit einer sich ändernden Farbe und/oder Farbtemperatur der Beleuchtung, insbesondere anhand eines auf der Anzeige dargestellten Farb-Scans.

In einer möglichen Ausgestaltung der vorliegenden Erfindung erfolgt eine Auswertung der aufgenommenen Bilder in mindestens einem Betriebsmodus mittels der Steuerung so, dass Informationen aus mindestens zwei bei Beleuchtungen unterschiedlicher Farbe und/oder Farbtemperatur aufgenommenen Bildern in ein Ergebnisbild integriert werden.

In einer möglichen Ausgestaltung der vorliegenden Erfindung wird das Ergebnisbild in mindestens einem Betriebsmodus auf einer Anzeige dargestellt.

In einer möglichen Ausgestaltung der vorliegenden Erfindung erfolgt die Auswertung der Bilder in mindestens einem Betriebsmodus mittels einer Strukturerfassung.

Bevorzugt erfolgt eine Auswertung von mindestens zwei bei Beleuchtungen unterschiedlicher Farbe und/oder Farbtemperatur aufgenommenen Bildern des beleuchteten Bereichs so, dass mittels der Strukturerfassung Informationen aus mindestens zwei bei Beleuchtungen unterschiedlicher Farbe und/oder Farbtemperatur aufgenommenen Bildern in ein Ergebnisbild integriert werden.

Insbesondere können so die zwei oder mehr bei unterschiedlicher Farbe und/oder Farbtemperatur aufgenommenen Bilder zunächst einzeln ausgewertet und bei der jeweiligen Farbe und/oder Farbtemperatur des Bildes erkennbare Strukturen aus dem Bild extrahiert und in dem Ergebnisbild in Kombination dargestellt werden.

In einer möglichen Ausgestaltung der vorliegenden Erfindung erfolgt eine Auswertung von mindestens drei bei Beleuchtungen unterschiedlicher Farbe und/oder Farbtemperatur aufgenommenen Bildern des beleuchteten Bereichs.

Bei diesen Ausgestaltungen erfolgt daher eine separate Auswertung der bei unterschiedlicher Farbe und/oder Farbtemperatur aufgenommenen Bilder, wobei das Ergebnis der Auswertung in einem gemeinsamen Ergebnisbild dargestellt wird.

In einigen der zuvor beschrieben Ausgestaltungen wurde dagegen ein Bild, welches bei einer anderen Farbe und/oder Farbtemperatur der Beleuchtung aufgenommen wurde, separat ausgegeben, insbesondere angezeigt.

In einer möglichen Ausgestaltung der vorliegenden Erfindung wird das separat ausgegebene und/oder ausgewertete Bild kontinuierlich aktualisiert.

In einer möglichen Ausgestaltung der vorliegenden Erfindung wird das separat ausgegebene und/oder ausgewertete Bild zu durch die Steuerung vorgegebenen Zeitpunkten aktualisiert.

In einer möglichen Ausgestaltung der vorliegenden Erfindung wird das separat ausgegebene und/oder ausgewertete Bild auf Grundlage eines durch eine manuelle Eingabe einer Bedienperson verursachten Signals erstellt und/oder aktualisiert.

Diese drei Varianten zur Erstellung und/oder Aktualisierung stehen in gleicher Weise auch für die Erstellung und/oder Darstellung eines aus bei mehreren unterschiedlichen Farben und/oder Farbtemperaturen der Beleuchtung aufgenommenen Bildern erstellten Ergebnisbildes zur Verfügung.

Bevorzugt kann die Beleuchtungsanordnung eine Eingabevorrichtung mit einem Fußschalter umfassen, wobei das separat ausgegebene und/oder ausgewertete Bild auf Grundlage eines durch eine Betätigung des Fußschalters verursachten Signals erstellt und/oder aktualisiert wird.

In einer möglichen Ausgestaltung der vorliegenden Erfindung handelt es sich bei mindestens einer Kamera um eine 2D-Kamera, zur Aufnahme eines 2D-Bildes des beleuchteten Bereichs.

In einer möglichen Ausgestaltung der vorliegenden Erfindung handelt es sich bei mindestens einer Kamera um eine 3D-Kamera, zur Aufnahme eines 3D-Bildes des beleuchteten Bereichs. Insbesondere kann die 3D-Kamera zwei Einzel-Kameras umfassen, welche eine Stereo-Sicht auf den beleuchteten Bereich erlauben.

In einer möglichen Ausgestaltung der vorliegenden Erfindung arbeitet mindestens eine Kamera in mindestens einem aus VIS-Bereich, IR-Bereich und/oder UV-Bereich.

Bevorzugt arbeitet die mindestens eine Kamera im VIS-Bereich, d.h. in einem Bereich des sichtbaren Lichts.

In einer möglichen Ausgestaltung der vorliegenden Erfindung handelt es sich bei der Beleuchtungsanordnung um eine Leuchtenanordnung.

In einer möglichen Ausgestaltung der vorliegenden Erfindung handelt es sich bei der mindestens einen Lichtquelle um eine Leuchte, insbesondere um eine Operationsleuchte.

Die Operationsleuchte kann einen Lichtquellenkörper mit einem mittig und/oder seitlich angeordneten Griff umfassen, durch welche die Position und/oder Ausrichtung der Operationsleuchte veränderbar ist.

Die Operationsleuchte kann an einem Tragesystem angeordnet sein, insbesondere an einem Tragesystem, welches an der Decke montiert ist. Insbesondere kann das Tragesystem eine Zentralwelle umfassen, an welcher einer oder mehrere Tragarme um eine vertikale Achse verschenkbar angeordnet sind, wobei die Operationsleuchte und/oder Operationsleuchten jeweils an einem Tragarm angeordnet sind.

In einer möglichen Ausgestaltung der vorliegenden Erfindung handelt es sich bei der mindestens einen Lichtquelle um eine Endoskopie-Lichtquelle.

In einer möglichen Ausgestaltung der vorliegenden Erfindung weist die Beleuchtungsanordnung mindestens einen Betriebsmodus auf, in welchem mittels einer Endoskopie-Kamera aufgenommene Bilder eines Bereichs, der mittels der Endoskopie-Lichtquelle beleuchtet ist, mittels der Steuerung auf einer Anzeige darstellbar sind.

Insbesondere kann die erfindungsgemäße Beleuchtungsanordnung daher bei endoskopischen Eingriffen zum Einsatz kommen, um den endoskopisch behandelten Bereich zu beleuchten.

In einer möglichen Ausgestaltung umfasst die Beleuchtungsanordnung daher neben der Lichtquelle einen Lichtleiter, über welchen das Licht der Lichtquelle zur Operationsstelle geleitet wird. Weiterhin umfasst die Beleuchtungsanordnung bevorzugt einen Lichtleiter, über welchen ein Bild der Operationsstelle zur Kamera geleitet wird. Hierbei können für die Lichtquelle und die Kamera getrennte Lichtleiter oder ein gemeinsamer Lichtleiter zum Einsatz kommen.

Bevorzugt verlaufen der oder die Lichtleiter durch ein endoskopisches Instrument, über welches sie in den Körper des Patienten eingeführt werden können.

Die Anzeige dient bei einer endoskopischen Beleuchtungsanordnung dem Operateur zur Beobachtung der Operationsstelle. Es kann sich hierbei um einen Monitor, eine Daten-, 3D- und/oder VR-Brille oder eine andere geeignete Anzeige handeln. In einer möglichen Ausgestaltung ermöglicht die Anzeige dem Operateur eine 3D-Sicht auf die Operationsstelle. Dies kann beispielsweise durch den Einsatz von zwei Kameras und/oder zwei Anzeigen erfolgen.

In einer möglichen Ausgestaltung der vorliegenden Erfindung sind einzelne bei jeweils unterschiedlicher Farbe und/oder Farbtemperatur aufgenommene Bilder der Endoskopie-Kamera mittels der Steuerung in ein Ergebnisbild ineinander integrierbar.

Bevorzugt handelt es sich bei dem Ergebnisbild um ein 2D-Ergebnisbild oder um ein 3D-Ergebnisbild.

Die vorliegende Erfindung umfasst weiterhin ein Verfahren zum Betreiben einer Beleuchtungsanordnung. Die Beleuchtungsanordnung umfasst hierbei mindestens eine Lichtquelle, mindestens eine Kamera und eine Steuerung. Erfindungsgemäß umfasst das Verfahren ein Verändern der Farbe und/oder Farbtemperatur der Lichtquelle und die Aufnahme von Bildern eines durch die Lichtquelle beleuchteten Bereiches mittels der Kamera. Das Verfahren umfasst weiterhin ein Ansteuern der Beleuchtungsanordnung mittels der Steuerung, sodass die Lichtquelle den beleuchteten Bereich nacheinander mit Licht unterschiedlicher Farbe und/oder Farbtemperatur beleuchtet, und ein separates Auswerten und/oder Ausgeben, insbesondere Darstellen, von mindestens einem ersten, bei einer ersten Farbe und/oder Farbtemperatur der Beleuchtung aufgenommenem Bildes.

Das Verfahren erfolgt bevorzugt so, wie dies oben im Hinblick auf die erfindungsgemäße Beleuchtungsanordnung bereits beschrieben wurde und/oder wie dies nachfolgend anhand der Figur sowie den Ausführungsbeispielen beschrieben wird.

Die vorliegende Erfindung umfasst weiterhin eine Software mit Befehlen zur Durchführung eines Verfahrens, wie es soeben dargestellt wurde.

Gemäß einer bevorzugten Ausgestaltung dient die Software weiterhin zur Integration von Informationen aus mindestens zwei bei Beleuchtungen unterschiedlicher Farbe und/oder Farbtemperatur aufgenommenen Bildern in ein Ergebnisbild.

Die Software ist bevorzugt so ausgestaltet, wie dies oben im Hinblick auf die erfindungsgemäße Beleuchtungsanordnung und/oder im Hinblick auf das Verfahren bereits beschrieben wurde und/oder wie dies nachfolgend anhand der Figur sowie den Ausführungsbeispielen beschrieben wird.

In einer möglichen Ausgestaltung der vorliegenden Erfindung umfasst die Steuerung der Beleuchtungsanordnung einen Mikrocontroller und einen nicht-flüchtigen Speicher, wobei in dem nicht-flüchtigen Speicher ein Programm mit Befehlen abgespeichert ist, welches, läuft es auf dem Microcontroller ab, ein oben oder im folgenden beschriebenes Verfahren durchführt und/oder einen oben oder im folgenden beschriebenen Betrieb der Lichtquellenanorndnung bewirkt. Insbesondere steuert der Microcontroller dabei die Lichtquelle und/oder der Anzeige an und/oder erhält Bilder der Kamera. Bei dem Microcontroller kann es sich um den Prozessor eines Computer handeln.

Die Anzeige, welche gemäß den oben beschriebenen Ausgestaltungen der vorliegenden Erfindung zum Einsatz kommt, umfasst bevorzugt um ein flächiges Anzeigeelement, welches zweidimensionale Bilder anzeigt.

In einer möglichen Ausgestaltung der vorliegenden Erfindung handelt es sich bei der Anzeige um einen Monitor. Dieser kann beispielsweise an geeigneter Position im oder außerhalb des Operationsraums angeordnet sein.

In einer möglichen Ausgestaltung der vorliegenden Erfindung handelt es sich bei der Anzeige um eine Daten-, 3D- und/oder VR-Brille.

Der Einsatz der vorliegenden Erfindung ist nicht auf den Bereich der Leuchten oder der Operationsleuchten beschränkt. Vielmehr kann die erfindungsgemäße Beleuchtungsanordnung auch bei der Materialbetrachtung und/oder Materialprüfung zum Einsatz kommen.

Wird im Rahmen der vorliegenden Erfindung von Beleuchtung gesprochen, ist dies nicht auf den Bereich des sichtbaren Lichts beschränkt. Vielmehr kann die vorliegende Erfindung mit elektromagnetischer Strahlung in jedem Spektralbereich eingesetzt werden.

Wird im Rahmen der vorliegenden Erfindung von einer Beleuchtung mit unterschiedlicher Farbe und/oder Farbtemperatur gesprochen, ist daher ganz allgemein Strahlung mit unterschiedlichem Spektrum gemeint.

Die Lichtquelle stellt daher eine Strahlungsquelle dar, welche so angesteuert werden kann, dass sie Strahlung mit unterschiedlichem Spektrum abgeben kann.

Hierfür kann die Strahlungsquelle beispielsweise auch zwei oder mehr Strahlungselemente umfassen, welche Strahlung mit einem zueinander unterschiedlichem Spektrum abgeben, und welche getrennt ansteuerbar sind.

Wird im Rahmen der vorliegenden Erfindung von einer Kamera gesprochen, so ist damit jede Sensoranordnung gemeint, die entsprechende Strahlung flächig und/oder räumlich detektieren kann. Bevorzugt umfasst die Kamera jedoch einen flächigen Sensorchip und/oder eine Optik, durch welche ein Bild auf den Sensorchip abgebildet wird. Der Sensorchip umfasst bevorzugt mehrere Farbkanäle, so dass die Kamera Farbbilder aufnehmen kann.

Insbesondere kann es sich bei der Kamera um eine Videokamera handeln.

In einer möglichen Ausgestaltung der vorliegenden Erfindung kommt eine TeraHertz-Kamera zum Einsatz.

Die vorliegende Erfindung wird nun anhand einer Zeichnung sowie Ausführungsbeispielen näher erläutert.

Dabei zeigt die:
- Figur: ein Ausführungsbeispiel der Beleuchtungsanordnung mit zwei Lichtquellen, wobei an mindestens einer der Lichtquellen eine Kamera angeordnet ist.

Die Figur zeigt ein Ausführungsbeispiel einer erfindungsgemäßen Beleuchtungsanordnung 1 mit einer ersten Lichtquelle 2 und einer zweiten Lichtquelle 2'. Die Beleuchtungsanordnung 1 könnte im Rahmen der vorliegenden Erfindung jedoch auch nur eine Lichtquelle 2 oder mehr als zwei Lichtquellen 2 umfassen.

Wie oben bereits erwähnt kann die vorliegende Erfindung in unterschiedlichen technischen Gebieten zum Einsatz kommen. Der Bezug zum Operationsumfeld bzw. medizinischen Umfeld hat lediglich beispielhaften Charakter.

Im Ausführungsbeispiel handelt es sich bei den Lichtquellen 2 und 2' jeweils um eine Operationsleuchte, wobei die Lichtquellen 2 und 2' jeweils ein Lichtfeld erzeugen, mittels welchen der Bereich B beleuchtet wird. Der Bereich B stellt im vorliegenden Ausführungsbeispiel einen Operationsbereich B dar.

Die Lichtquellen 2 und 2' können jeweils so ausgerichtet und/oder positioniert werden, dass der Operationsbereich B eines auf einem Tisch 8 liegenden Patienten P wie gewünscht beleuchtet wird. In anderen Fällen können die Lichtfelder der beiden Lichtquellen 2 und 2' auch auf unterschiedliche Bereiche ausgerichtet sein. Dies ist beispielsweise bei einer Transplantation denkbar, wobei eine Operationsleuchte 2 auf den Operationsbereich B gerichtet sein kann, während eine Operationsleuchte 2' auf das zu transplantierende Organ gerichtet sein kann.

Sind wie im Ausführungsbeispiel mehrere Lichtquellen 2 und 2' vorgesehen, kann die Beleuchtung des Bereichs B mittels einer der Lichtquellen 2 bzw. 2' oder mittels beiden Lichtquellen 2 und 2' erfolgen, wobei die Steuerung 20 die Lichtquellen 2 und 2' entsprechend ansteuert.

Im Ausführungsbeispiel ist ein Tragesystem 3 vorgesehen, an welchem die Lichtquellen 2 und 2' oberhalb des beleuchteten Bereichs B angeordnet sind. Ein Verstellen der Lichtquellen 2 und 2' kann manuell erfolgen. Des Weiteren kann eine Verstellung durch einen oder mehrere Antriebe des Tragesystems 3 realisiert werden.

Im Ausführungsbeispiel umfasst das Tragesystem 3 eine Deckenhalterung, an der eine Zentralwelle angeordnet sein kann. An der Zentralwelle wiederum können Tragarme um eine durch die Zentralwelle gebildete vertikale Achse verschwenkbar angeordnet sein. Die Lichtquellen 2 und 2' können jeweils über weitere Tragarmelemente, Federbügel und/oder eine Kardanik mit einem oder mehreren Gelenken 5, 6 an unterschiedlichen Tragarmen angeordnet sein. Andere Ausgestaltungen des Tragesystems 3 als die soeben beschriebene sind ebenso denkbar.

Im Ausführungsbeispiel umfasst die Beleuchtungsanordnung 1 mindestens eine Kameras 10 und/oder 15. Die Beleuchtungsanordnung 1 kann im Rahmen der vorliegenden Erfindung nur eine Kamera 10, 15, zwei Kameras 10, 15 oder mehr als zwei Kameras umfassen.

Im Ausführungsbeispiel ist die Kameras 10,15 jeweils an einer Lichtquelle 2, 2' angeordnet und ist insbesondere in einen Griff 7, 7' einer Kamera 10, 15 integriert sein. Bevorzugt ist der Griff 7, 7' mittig an einem Lichtquellenkörper der Lichtquelle 2, 2' angeordnet.

Insbesondere weist die Kamera 10, 15 eine optische Hauptachse auf, welche parallel zur optischen Hauptachse der Lichtquelle, an welcher sie angeordnet ist, verläuft, wobei bevorzugt die optische Hauptachse der Kamera mit der optischen Hauptachse der Lichtquelle zusammenfällt.

Es ist auch denkbar, eine oder mehrere Kameras 10 und 15 am Tragesystem 3 vorzusehen.

Des Weiteren ist im Ausführungsbeispiel ein Monitor 60 zur Anzeige eines mittels der Kamera 10, 15 aufgenommenen Bildes 11 vorgesehen, wobei der Monitor 60 wie dargestellt über ein Tragarmelement 6 am Tragesystem 3 angeordnet sein kann. Im Ausführungsbeispiel befindet sich der Monitor 60 im Operationsraum. Es ist allerdings auch denkbar, dass der Monitor 60 in einem vom Operationsraum separaten Raum vorgesehen ist. Somit kann das Bild 11 der Kamera 10, 15 beispielsweise zu Schulungszwecken zur Verfügung gestellt werden. Es ist auch denkbar, dass ein Bild einer Kamera zu Dokumentationszwecken einer Speichereinheit zugeführt wird.

Der Begriff "Bild" im Sinne der vorliegenden Erfindung umfasst sowohl ein Videobild im Sinne einer Bildsequenz als auch ein einzelnes Bild im Sinne eines Fotos und/oder ein einzelnes Bild aus einer Bildsequenz im Sinne eines Frames einer Videobildsequenz.

In einer möglichen Ausgestaltung der vorliegenden Erfindung handelt es sich bei dem Bild um ein Videobild und/oder einen Frame einer Videobildsequenz.

Im Ausführungsbeispiel ist weiterhin eine manuell bedienbare Eingabevorrichtung 40 vorgesehen, mittels welcher eine für das menschliche Auge wahrnehmbare Hauptbeleuchtungs-Farbe und/oder Farbtemperatur manuell eingestellt werden kann. Wie dargestellt kann die Eingabevorrichtung 40 mehrere Eingabeelemente 41 aufweisen, mittels welchen eine gewünschte Hauptbeleuchtungs-Farbe und/oder Farbtemperatur und/oder ein Betriebsmodus der Beleuchtungsanordnung 1 ausgewählt werden kann. Die Eingabeelemente 41 können beispielsweise in Form von Schaltern, Reglern und/oder eines Touchscreens vorliegen. Beispielsweise kann ein erstes Eingabeelement 41 zum Einstellen eines oder mehrerer Betriebsmodi der Beleuchtungsanordnung 1 vorgesehen sein, ein zweites Eingabeelement 41 zum Einstellen einer Hauptbeleuchtungs-Farbe und/oder Hauptbeleuchtungs-Farbtemperatur der Beleuchtung und/oder ein drittes Eingabeelement zum Einstellen einer weiteren Farbe und/oder Farbtemperatur der Beleuchtung, bei welcher ein Bild separat ausgewertet und/oder ausgegeben wird, vorgesehen sein.

Die Beleuchtungsanordnung 1 umfasst wie in der Figur dargestellt eine Steuerung 20. Die Steuerung 20 kann kabelgebunden und/oder kabellos mit mindestens einer der Lichtquellen 2 und 2' und/oder mit der Eingabevorrichtung 40 vernetzt sein. Es ist auch denkbar, dass die Lichtquellen 2 und 2' miteinander kabellos und/oder kabelgebunden vernetzt sind. Weiterhin kann die Steuerung in eine der Lichtquellen und/oder die Eingabevorrichtung integriert sein.

In einem ersten Aspekt der vorliegenden Erfindung steuert die Steuerung 20 die Lichtquellen so an, dass diese den beleuchteten Bereich B nacheinander mit Licht unterschiedlicher Farbe und/oder Farbtemperatur beleuchten, wobei die Steuerung 20 mindestens ein erstes, bei einer ersten Farbe und/oder Farbtemperatur der Beleuchtung aufgenommenes Bild 11 separat auswertet und/oder ausgibt.

Anhand eines Beispiels wird nun ein möglicher Betrieb der in der Figur dargestellten Beleuchtungsanordnung 1 erläutert:

Die Lichtquelle 2 und/oder Lichtquelle 2' beleuchtet den Bereich in einer ersten Ausführungsform mit weißem Licht mit einer einstellbaren Hauptbeleuchtungs-Farbtemperatur, beispielsweise von 4.000 K. Diese Hauptbeleuchtungs-Farbtemperatur kann mittels der Eingabevorrichtung 40 manuell eingestellt werden. Alternativ kann die Lichtquelle 2, 2' den Bereich mit farbigem Licht beleuchten, wobei eine Hauptfarbe der Beleuchtung einstellbar ist, beispielsweise blaues Licht mit einem Wellenlängenbereich zwischen beispielsweise 380 und 500 Nanometern.

Der Betrieb der Kamera 10 bzw. 15 zur Aufnahme von Bildern 11 bis 14 des beleuchteten Bereichs kann bei einer vorgegebenen Frequenz, beispielsweise von 50 Hz erfolgen, sodass die Kamera beispielsweise jeweils 20 ms lang ein Bild des beleuchteten Bereichs B aufnimmt.

Die Beleuchtungsanordnung 1 weist einen Betriebsmodus auf, in dem die Steuerung 20 die Beleuchtungsanordnung 1 derart ansteuert, dass die Lichtquelle 2, 2' den beleuchteten Bereich B nacheinander mit Licht unterschiedlicher Farbtemperatur, beispielsweise zu Werten von 3.000 K, 3.500 K, 4.000 K, 5.000 K, 5.500 K, beleuchtet.

In einer möglichen Ausgestaltung der vorliegenden Erfindung kann eine jeweilige Beleuchtungsdauer mit dem entsprechenden Farbtemperaturwert jeweils einen festen Wert betragen, beispielsweise 32 ms.

In einer bevorzugten Ausführungsform wechselt die Beleuchtung jeweils von einer Hauptbeleuchtungs-Farbtemperatur zu einer der Farbtemperaturen und zurück, bevor zur nächsten Farbtemperatur gewechselt wird.

Die Beleuchtungsanordnung und/oder Kamera 10 bzw. 15 kann nun so angesteuert werden, dass jeweils während jeder der Beleuchtungen mit einem der unterschiedlichen Farbtemperaturwerte mindestens ein Bild 11 aufgenommen wird.

So können mehrere bei jeweils unterschiedlicher Farbtemperatur aufgenommene Bilder aufgenommen werden, und/oder mindestens ein bei einer anderen als der Hauptbeleuchtungs-Farbtemperatur aufgenommenes Bild.

Das oder die Bilder unterschiedlicher Farbtemperatur können sodann auf dem Monitor 60 angezeigt werden. Werden mehrere Bilder, welche mit einer Beleuchtung mit unterschiedlicher Farbtemperatur aufgenomen wurden, angezeigt, kann dies nacheinander und/oder gleichzeitig erfolgen, beispielsweise als Bild im Bild.

Alternativ oder zusätzlich kann eine Auswertung der Bilder derart erfolgen, dass Informationen aus mindestens zwei der bei unterschiedlicher Farbtemperatur aufgenommenen Bilder 11 mittels der Steuerung 20 ausgewertet in einem Ergebnisbild 14 kombiniert werden. So können in dem Ergebnisbild 14 Strukturunterschiede des beleuchteten Bereichs gut sichtbar gemacht werden, die sonst nur auf separaten Bildern gut sichtbar wären. Insbesondere können durch die Steuerung in den einzelnen Bildern Strukturen erfasst und in einem Ergebnisbild kombiniert werden.

Das Ergebnisbild 14 kann beispielsweise auf dem in der Figur gezeigten Monitor 60 dargestellt werden, sodass dieses dem Operateur O während der Operation zur Verfügung gestellt werden kann.

Die Steuerung 20 kann mindestens einen Betriebsmodus aufweisen, in welchem sie die Beleuchtungsanordnung 1 derart ansteuert, dass die Beleuchtung des Bereichs mit den unterschiedlichen Farbtemperaturwerten derart schnell hintereinander erfolgt, dass dies aufgrund der Trägheit des menschlichen Auges vom menschlichen Auge bei unmittelbarer Betrachtung des beleuchteten Bereichs nicht wahrgenommen werden kann.

Im Ausführungsbeispiel erfolgt die Ansteuerung daher so, dass sich eine von dem Operateur O bei unmittelbarer Betrachtung des Bereichs B wahrgenommene Hauptbeleuchtungs-Farbtemperatur auch während der erfindungsgemäßen Veränderung der Farbtemperatur der Beleuchtung nicht ändert.

Durch die separate Auswertung und/oder Ausgabe von Bildern, welche bei den durch das Auge nicht wahrnehmbaren Farbtemperaturen aufgenommen wurden, können dem Operateur zusätzliche Informationen, insbesondere in Form von einem oder mehreren Bildern mit einer anderen Farbtemperatur, zur Verfügung gestellt werden.

Alternativ oder zusätzlich zu der zuvor beschriebenen Veränderung der Farbtemperatur der Beleuchtung kann - bei ansonsten unveränderter Vorgehensweise - eine Veränderung der Farbe der Beleuchtung erfolgen.

Weitere mögliche Ausgestaltungen der vorliegenden Erfindung, insbesondere mögliche Betriebsmodi der Beleuchtungsanordnung 1, werden im folgenden beschrieben.

In einem ersten Betriebsmodus beleuchtet die Lichtquelle 2 den Bereich B mit einer Hauptbeleuchtungs-Farbe und/oder Farbtemperatur, wobei auf dem Monitor 60 ein bei einer ersten Farbe und/oder Farbtemperatur aufgenommenes Bild 11 des beleuchteten Bereichs B dargestellt ist. Die erste Farbe und/oder Farbtemperatur des Bildes 11 unterscheidet sich von der Hauptbeleuchtungs-Farbe und/oder Farbtemperatur. Dies ermöglicht es, dass bei Betrachtung des Bildes 11 und bei unmittelbarer Betrachtung des beleuchteten Bereichs B jeweils unterschiedliche Strukturen gut sichtbar gemacht werden können. Insbesondere wechselt die Steuerung die Farbe und/oder Farbtemperatur der Beleuchtung von der Hauptbeleuchtungs-Farbe und/oder Farbtemperatur kurzzeitig zur ersten Farbe und/oder Farbtemperatur, wobei ein in diesem Zeitraum aufgenommenes Bild auf dem Monitor 60 dargestellt wird.

In einem zweiten Betriebsmodus wird auf dem Monitor 60 ein weiteres Bild 12 der Kamera 10 dargestellt, wobei das Bild 12 bei einer zweiten Farbe und/oder Farbtemperatur der Beleuchtung aufgenommen wurde. Die zweite Farbe und/oder Farbtemperatur unterscheidet sich von der ersten Farbe und/oder Farbtemperatur des Bildes 11. Anhand dieser dargestellten Bilder 11 und 12 jeweils unterschiedlicher Farbe und/oder Farbtemperatur können dem Operateur O jeweils unterschiedliche Strukturen gut sichtbar gemacht werden. Es ist beispielsweise denkbar, dass auf dem Bild 11 der Unterschied zwischen einem Tumorgewebe und einem gesunden Gewebe gut sichtbar ist, während auf dem Bild 12 der Unterschied zwischen Muskelgewebe und Nervengewebe gut sichtbar ist. Insbesondere wechselt die Steuerung hierfür die Farbe und/oder Farbtemperatur der Beleuchtung von der Hauptbeleuchtungs-Farbe und/oder Farbtemperatur jeweils kurzzeitig zur ersten Farbe und/oder Farbtemperatur und zur zweiten Farbe und/oder Farbtemperatur, wobei in den jeweiligen Zeiträumen aufgenommene Bilder auf dem Monitor 60 dargestellt werden.

In einem dritten Betriebsmodus wird ein Bild 13 des beleuchteten Bereichs B auf dem Monitor 60 dargestellt, welches den beleuchteten Bereich B bei einer sich in seiner Farbe und/oder Farbtemperatur ändernden Beleuchtung zeigt. Dies ermöglicht es dem Operateur O, eine geeignete Farbe und/oder Farbtemperatur relativ kurzfristig zu ermitteln. Auf Basis dieser Ermittlung könnte die Hauptbeleuchtungs-Farbe und/oder Farbtemperatur geändert werden.

Weiterhin kann auch die erste und/oder zweite Farbe und/oder Farbtemperatur einstellbar sein.

In einem vierten Betriebsmodus wird auf dem Monitor ein Ergebnisbild angezeigt, welches Informationen aus mindestens zwei Bildern umfasst, welche bei Beleuchtungen unterschiedlicher Farbe und/oder Farbtemperatur aufgenommenen werden.

Die Steuerung 20 umfasst hierfür bevorzugt eine Strukturerfassung 30, wobei die Strukturerfassung 30 in die Steuerung 20 integriert sein kann. Es ist auch denkbar, dass die Strukturerfassung 30 separat zur Steuerung 20 vorgesehen ist.

In einer möglichen Ausgestaltung der vorliegenden Erfindung handelt es sich bei der Strukturerfassung um eine Strukturerkennungssoftware.

Die Strukturerfassung 30 dient der Erfassung von Strukturen in aufgenommenen Bildern. Die in den bei unterschiedlicher Farbe und/oder Farbtemperatur der Beleuchtung aufgenommenen Bildern jeweils erfassten Strukturen werden bevorzugt in einem Ergebnisbild kombiniert dargestellt.

Beispielsweise kann mittels der Strukturerfassung 30 in einem ersten bei einer ersten Farbe und/oder Farbtemperatur aufgenommenen Bild 11 ein gut sichtbarer Kontrastunterschied zwischen einem gesunden Gewebe und einem Tumorgewebe identifiziert werden, wobei mittels der Strukturerfassung 30 in einem zweiten bei einer zweiten Farbe und/oder Farbtemperatur aufgenommenen Bild 11 ein gut sichtbarer Kontrastunterschied zwischen einem Nervengewebe und einem Muskelgewebe identifiziert werden kann. Entsprechend kann die Steuerung 20 mittels der Strukturerfassung 30 auf Basis von Informationen aus dem ersten und dem zweiten Bild ein Ergebnisbild erzeugen, in welchen sowohl der gut sichtbare Unterschied zwischen Tumorgewebe und gesundem Gewebe aufgrund der Beleuchtung mit der ersten Farbe und/oder Farbtemperatur als auch der gut sichtbare Unterschied zwischen Nervengewebe und Muskelgewebe aufgrund der Beleuchtung mit der zweiten Farbe und/oder Farbtemperatur sichtbar ist.

In einer möglichen Ausgestaltung der vorliegenden Erfindung kann eine Recheneinheit 50 vorgesehen sein, mittels welcher eine Integration von Informationen aus mindestens zwei bei Beleuchtungen unterschiedlicher Farbe und/oder Farbtemperatur aufgenommenen Bildern in ein Ergebnisbild erfolgen kann, wobei vorzugsweise für diese Integration die Strukturerfassung 30 herangezogen wird.

Die Recheneinheit 50 kann zusammen mit der Strukturerfassung 30 in die Steuerung 20 integriert sein. Es ist auch denkbar, dass die Recheneinheit 50 separat zur Steuerung 20 vorgesehen ist. Die Recheneinheit 50 kann kabelgebunden und/oder kabellos mit der Steuerung 20 und/oder mit der Strukturerfassung 30 vernetzt sein.

In einer möglichen Ausgestaltung der vorliegenden Erfindung umfasst die Beleuchtungsanordnung mindestens zwei Betriebsmodi. Bevorzugt ist die Steuerung so ausgeführt, dass eine Bedienperson mindestens einen Betriebsmodus und/oder mehrere Betriebsmodi auswählen kann. Insbesondere können mehrere der oben beschriebenen ersten bis vierten Betriebsmodi zur Verfügung stehen, wobei eine Bedienperson einen oder mehrere aus diesen auswählen kann. Bevorzugt erfolgt diese Auswahl mittels einer manuell betätigbaren Eingabevorrichtung 40. Die Beleuchtungsanordnung 1 kann gleichzeitig in mehreren Betriebsmodi arbeiten.

Die Aktualisierung des angezeigten Bildes kann kontinuierlich und/oder in vorgegebenen Zeitabständen erfolgen. Insbesondere kann hierfür kontinuierlich und/oder in vorgegebenen Zeitabständen ein Wechsel zu mindestens einer anderen Farbe und/oder Farbtemperatur und/oder ein Farb- bzw. Farbtemperatur-Scan durchgeführt werden.

Alternativ oder zusätzlich kann die Aktualisierung des angezeigten Bildes durch einen Bediener getriggert werden, insbesondere durch Betätigen einer Eingabevorrichtung.

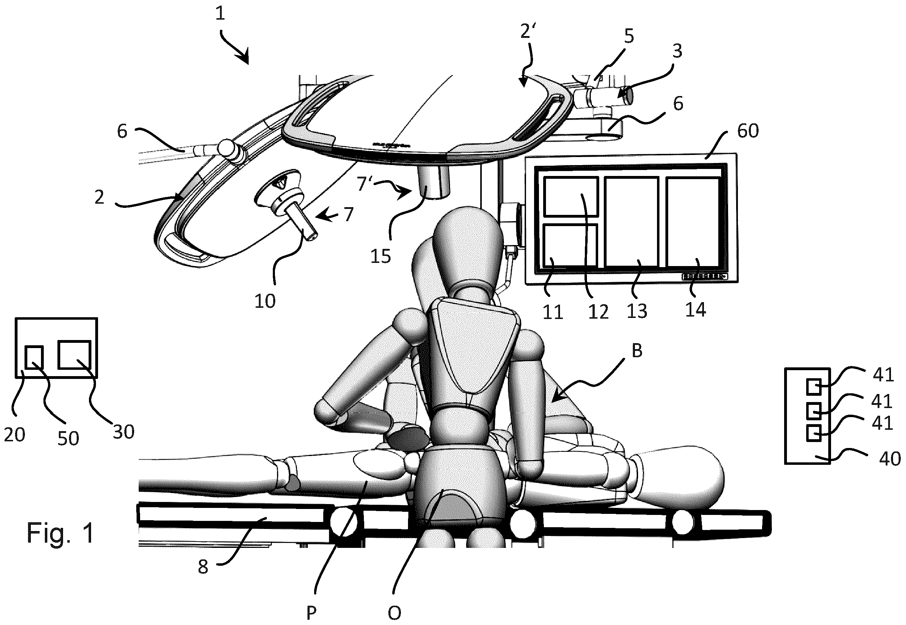

In dem in Fig. 1 dargestellten Ausführungsbeispiel handelt es sich bei der Beleuchtungsanordnung um eine Operationsleuchtenanordnung. Die vorliegende Erfindung ist hierauf jedoch nicht beschränkt und kann auch bei beliebigen anderen Lichtquellen zum Einsatz kommen, insbesondere bei allen Beleuchtungsanwendungen in einem OP.

In einer möglichen Ausgestaltung der vorliegenden Erfindung handelt es sich bei der mindestens einen Lichtquelle um eine Endoskopie-Lichtquelle und/oder bei der mindestens einen Kamera um eine Endoskopie-Kamera. Hier kann die vorliegende Erfindung in gleicher Weise eingesetzt werden, wie dies oben im Hinblick auf das in Fig. 1 beschriebene Ausführungsbeispiel beschrieben ist.

Informationen aus bei jeweils unterschiedlicher Farbe und/oder Farbtemperatur der Beleuchtung mittels der Endoskopie-Kamera aufgenommenen Bildern können dabei mittels der Steuerung 20 in ein Ergebnisbild 14 integriert werden. Das Ergebnisbild 14 kann auf dem Monitor 60 dargestellt werden, wobei das Ergebnisbild 14 bevorzugt kontinuierlich und/oder zu durch die Steuerung vorgegebenen Zeitpunkten aktualisiert wird.

Kommt die vorliegende Erfindung im Bereich der Endoskopie zum Einsatz, muss auf den unmittelbaren optischen Eindruck der Beleuchtung keine Rücksicht mehr genommen werden, da der Operateur O ohnehin nur das Videobild sieht. Bevorzugt kann in diesem Fall eine stärkere Änderung der Farbe und/oder Farbtemperatur der Beleuchtung erfolgen oder längere Phasen für die einzelnen Farben und/oder Farbtemperaturen bei der Änderung der Beleuchtung gewählt werden, beispielsweise im Vergleich zu einem Einsatz der vorliegenden Erfindung bei einer Operationsleuchte. Im Bereich der Endoskopie ist es allerdings von Bedeutung, dass die Bilder zum Einen verzögerungsfrei wiedergegeben werden und zum Anderen eine flüssige Bilddarstellung erfolgt.

Unabhängig von der sonstigen Ausgestaltung der Beleuchtungsanordnung kann es sich bei der Kamera kann es sich zunächst um eine 2D-Kamera handeln.

Die vorliegende Erfindung kann jedoch auch dann eingesetzt werden, wenn es sich bei der Kamera um eine 3D-Kamera handelt. In diesem Fall handelt es sich bei den oben beschriebenen Bildern um 3D-Bilder. Die Ausgabe kann in diesem Fall über einen Monitor mit 3D-Darstellung und/oder eine 3D-Brille erfolgen.

Eine 3D-Kamera kann zwei Einzelkameras umfassen, welche in Form einer Stereo-Kamera zusammenwirken.

Zudem wäre es denkbar, Informationen eines Bildes 11 einer ersten Kamera 10 einer ersten Operationsleuchte mit Informationen eines Bildes einer zweiten Kamera 15 einer zweiten Operationsleuchte zur Erzeugung eines 3D-Bildes zu verwenden. Dies kann beispielsweise mittels einer Recheneinheit 50 erfolgen.

In einem bevorzugten Ausführungsbeispiel handelt es sich bei der Lichtquelle 2 um eine LED-Lichtquelle 2. Im Ausführungsbeispiel werden die LEDs der LED-Lichtquelle 2 mittels der Steuerung gepulst betrieben. In einem solchen gepulsten Betrieb sind die LEDs entweder ausgeschaltet oder für die Dauer eines Pulses eingeschaltet, wobei die Farbe und/oder Farbtemperatur sowie die Helligkeit der Lichtquelle 2 über die Dauer der einzelnen Pulse, in welchen die LEDs eingeschaltet sind, angesteuert wird. Beispielsweise kann der gepulste Betrieb bei einer Frequenz von 300 Hz erfolgen.

Insbesondere umfasst die LED-Lichtquelle 2 mindestens zwei unterschiedliche Arten von LED zur Erzeugung von Licht unterschiedlicher Farbe und/oder Farbtemperatur. Durch die Länge der Pulse, mit welchen die jeweiligen LEDs betrieben werden, kann hierdurch die Steuerung die Farbe und/oder Farbtemperatur einstellen.

In einer ersten Ausführungsform umfasst die LED-Lichtquelle 2 zwei Arten von LEDs, welche weißes Licht mit einer unterschiedlichen Farbtemperatur erzeugen, beispielsweise einen ersten Typ LED mit einer Farbtemperatur von 2700 K oder 3000 K und einen zweiten Typ LED mit einer Farbtemperatur von 5000 K oder 5500 K, wobei die Steuerung durch die Ansteuerung des Verhältnisses der durch die beiden Typen von LEDs abgestrahlten Lichtmenge die Farbtemperatur auf einen gewünschten Wert einstellt.

In einer zweiten Ausführungsform umfasst die LED-Lichtquelle 2 zwei Arten von LEDs, welche Licht mit einer unterschiedlichen Farbe erzeugen, beispielsweise einen ersten Typ LED, welcher rotes Licht abstrahlt, und einen zweiten Typ LED, welcher blaues Licht abstrahlt, wobei die Steuerung durch die Ansteuerung des Verhältnisses der durch die beiden Typen von LEDs abgestrahlten Lichtmenge die Farbe und/oder Farbtemperatur auf einen gewünschten Wert einstellt. Die gepulste Ansteuerung der LED erlaubt es, die Farbe und/oder Farbtemperatur der Beleuchtung sehr schnell zu ändern. Insbesondere kann die Farbe und/oder Farbtemperatur für jeden Puls einzeln angesteuert werden.

Die vorliegenden Erfindung nutzt diese schnelle Ansteuerbarkeit der LEDs, um in einen Betreib der LEDs mit einer Hauptbeleuchtungs-Farbe und/oder Farbtemperatur einen oder mehrere Pulse mit einer anderen Farbe und/oder Farbtemperatur einzustreuen, welche aufgrund der Trägheit des menschlichen Auges für einen Betrachter nicht erkennbar sind.

Durch eine entsprechende Auswahl und/oder Auswertung der durch die Kamera aufgenommenen Bilder kann jedoch mindestens ein bei einer anderen als der Hauptbeleuchtungs-Farbe und/oder Farbtemperatur aufgenommenes Bild separat ausgewertet und/oder angezeigt werden.

Bei der Kamera handelt es sich bevorzugt um eine Video-Kamera, welche den beleuchteten Bereich mit einer gewissen Framerate aufnimmt, beispielsweise mit 60 Hz, d.h. 60 Bildern pro Sekunde.

Erfolgt die Beleuchtung durch die LEDs daher mit unterschiedlichen Farben und/oder Farbtemperaturen, so werden die Bilder bzw. Frames der Kamera jeweils ein Bild bei einer anderen Farbe und/oder Farbtemperatur der Beleuchtung zeigen. Erfindungsgemäß werden die bei unterschiedlicher Farbe und/oder Farbtemperatur aufgenommenen Bilder nun separat ausgewertet und/oder ausgegeben, bzw. angezeigt. Durch die Kamera kann die Beleuchtung daher in die einzelnen unterschiedlichen Farben und/oder Farbtemperaturen aufgelöst werden, während das menschliche Auge diese aufgrund seiner größeren Trägheit nicht wahrnimmt.

In einer möglichen Ausgestaltung erfolgt eine Synchronisation zwischen der Ansteuerung der LEDs und der Aufnahme durch die Kamera und/oder die Auswertung der Bilder der Kamera. Die Steuerung weiß daher, in welchen Zeitabschnitten eine Beleuchtung mit einer anderen Farbe und/oder Farbtemperatur erfolgte, und wertet die während dieses Zeitraums aufgenommenen Bilder separat aus. Weiterhin kann eine solche Synchronisation auch dazu dienen, um sicherzustellen, dass sich während der Aufnahme eines Bildes 11 die Farbe und/oder Farbtemperatur der Pulse des angesteuerten LEDs nicht ändert. Somit kann sichergestellt werden, dass ein Bild des beleuchteten Bereichs bei einer gewünschten Farbe und/oder Farbtemperatur der Beleuchtung aufgenommen wird, und nicht unterschiedliche Farben und/oder Farbtemperaturen der Beleuchtung in einem Bild vermischt werden.

Alternativ oder zusätzlich kann die Steuerung 20 die Beleuchtungsanordnung 1 so ansteuern, dass die Pulse der angesteuerten LEDs, die in einen Zeitabschnitt fallen, in dem ein Bild aufgenommen wird, im Mittel die gewünschte Farbe und/oder Farbtemperatur ergeben.

Auf eine solche Synchronisation kann jedoch auch verzichtet werden.

In einer möglichen Ausgestaltung der vorliegenden Erfindung wertet die Steuerung die Farbe und/oder Farbtemperatur der durch die Kamera aufgenommenen Bilder aus und erkennt anhand der Auswertung, bei welcher Farbe und/oder Farbtemperatur die Bilder jeweils aufgenommen wurden. Bilder, welche bei einer bestimmten Farbtemperatur und/oder Farbe aufgenommen wurden, werden dann separat ausgewertet und/oder ausgegeben.

Bevorzugt erfolgt die Beleuchtung mit einer Farbe und/oder Farbtemperatur in diesem Falle so, dass durch die Dauer der jeweiligen Beleuchtung sichergestellt ist, dass mindestens ein Bild aufgenommen wird, bei welchem zumindest für mehr als 50% der in das Bild eingehenden Aufnahmezeit mit einer gewünschten Farbe und/oder Farbtemperatur beleuchtet wurde und/oder für diese Aufnahmezeit eine gewünschte mittlere Farbe und/oder Farbtemperatur erzielt werden kann.

Wird beispielsweise die Beleuchtung mit einer anderen Farbe und/oder Farbtemperatur jeweils für eine Zeitdauer durchgeführt, welche der doppelten Dauer eines Frames entspricht, ist immer sichergestellt, dass mindestens ein Frame mit der gewünschten Farbe und/oder Farbtemperatur belichtet wird.

### Bezugszeichenliste:

- 1: Beleuchtungsanordnung
- 2, 2': Lichtquelle
- 3: Tragesystem
- 5: Tragarm
- 6: Tragarmelement
- 7: Griff
- 8: Operationstisch

- 10: Kamera
- 11: Bild (aufgenommen bei einer ersten Farbe und/oder Farbtemperatur)
- 12: Bild (aufgenommen bei einer zweiten Farbe und/oder Farbtemperatur)
- 13: Bild (ein sich in seiner Farbe und/oder Farbtemperatur änderndes Bild)
- 14: Ergebnisbild
- 15: Kamera

- 20: Steuerung
- 30: Strukturerfassung
- 40: Eingabevorrichtung
- 41: Eingabeelemente
- 50: Recheneinheit
- 60: Monitor

- B: Bereich
- O: Operateur
- P: Patient

## Patentansprüche

1. Beleuchtungsanordnung (1), welche mindestens eine Lichtquelle (2, 2') umfasst, mit mindestens einer Kamera (10, 15) und einer Steuerung (20), wobei die Farbe und/oder Farbtemperatur der Lichtquelle (2) veränderbar ist und die Kamera (10) Bilder (11, 12) eines durch die Lichtquelle (2) beleuchteten Bereichs (B) aufnimmt,
wobei die Steuerung (20) die Beleuchtungsanordnung (1) so ansteuert, dass die Lichtquelle (2) den beleuchteten Bereich (B) nacheinander mit Licht unterschiedlicher Farbe und/oder Farbtemperatur beleuchtet, wobei die Steuerung (20) mindestens ein erstes, bei einer ersten Farbe und/oder Farbtemperatur der Beleuchtung aufgenommenes Bild (11) separat auswertet und/oder ausgibt, **dadurch gekennzeichnet,**
**dass** die Steuerung (20) die Lichtquelle (2) in mindestens einem Betriebsmodus so ansteuert, dass die Veränderung der Farbe und/oder Farbtemperatur so schnell erfolgt, dass dies für das menschliche Auge bei unmittelbarer Betrachtung des beleuchteten Bereichs (B) nicht wahrnehmbar ist.

2. Beleuchtungsanordnung (1) nach Anspruch 1, wobei eine für das menschliche Auge wahrnehmbare Hauptbeleuchtungs-Farbe und/oder Farbtemperatur, mit der die Lichtquelle (2) den Bereich (B) beleuchtet, manuell einstellbar ist.

3. Beleuchtungsanordnung (1) nach einem der vorangegangenen Ansprüche, wobei die Lichtquelle (2) in einem Betriebsmodus so angesteuert wird, dass die Lichtquelle (2) den Bereich (B) mit einer Hauptbeleuchtungs-Farbe und/oder Farbtemperatur beleuchtet und von dieser Hauptbeleuchtungs-Farbe und/oder Farbtemperatur für einen Zeitabschnitt zur ersten Farbe und/oder Farbtemperatur wechselt, wobei während dieses Zeitabschnitts mindestens ein Bild (11) aufgenommen wird, welches separat ausgewertet und/oder ausgegeben wird, wobei der Zeitabschnitt bevorzugt so kurz ist, dass sich bei unmittelbarer Betrachtung des beleuchteten Bereichs (B) eine wahrgenommene Farbe und/oder Farbtemperatur durch den Wechsel nicht ändert, und insbesondere weniger als 200 ms, bevorzugt weniger als 50 ms lang ist.

4. Beleuchtungsanordnung (1) nach einem der vorangegangenen Ansprüche, wobei es sich bei der Lichtquelle (2) um eine LED-Lichtquelle (2) handelt, wobei die LEDs der LED-Lichtquelle (2) mittels der Steuerung (20) gepulst betrieben werden, wobei die Steuerung (20) die Beleuchtungsanordnung (1) in mindestens einem Betriebsmodus bevorzugt so ansteuert, dass sich während der Aufnahme des Bildes (11) die Farbe und/oder Farbtemperatur der Pulse nicht ändert und/oder dass die während der Aufnahme eines Bildes erfolgende Pulse im Mittel eine gewünschte Farbe und/oder Farbtemperatur ergeben.

5. Beleuchtungsanordnung (1) nach einem der vorangegangenen Ansprüche, wobei die Steuerung (20) die Beleuchtungsanordnung (1) in mindestens einem Betriebsmodus so ansteuert, dass die Beleuchtung nacheinander mit einer Mehrzahl von unterschiedlichen Farben und/oder Farbtemperaturen erfolgt, wobei die Steuerung (20) mehrere, bei jeweils unterschiedlichen Farben und/oder Farbtemperaturen der Beleuchtung aufgenommene Bilder separat auswertet und/oder ausgibt, wobei bevorzugt jeweils von einer Hauptbeleuchtungs-Farbe und/oder Farbtemperatur zu einer der unterschiedlichen Farben und/oder Farbtemperaturen und zurück gewechselt wird.

6. Beleuchtungsanordnung (1) nach einem der vorangegangen Ansprüche, wobei in einem Betriebsmodus auf einer Anzeige (60) der Beleuchtungsanordnung (1) ein Bild (11) dargestellt wird, welches den beleuchten Bereich (B) bei einer anderen Farbe und/oder Farbtemperatur der Beleuchtung zeigt als jene Farbe und/oder Farbtemperatur der Lichtquelle (2), die während der Darstellung des Bildes (11) bei unmittelbarer Betrachtung des beleuchteten Bereichs (B) wahrnehmbar ist.

7. Beleuchtungsanordnung (1) nach einem der vorangegangenen Ansprüche, wobei in einem Betriebsmodus auf einer Anzeige (60) der Beleuchtungsanordnung (1) mehrere Bilder (11, 12) dargestellt werden, welche den beleuchten Bereich (B) bei jeweils unterschiedlicher Farbe und/oder Farbtemperatur der Beleuchtung zeigen, und/oder wobei in einem Betriebsmodus auf der Anzeige (60) mindestens ein Bild (13) dargestellt wird, welches den beleuchteten Bereich (B) bei einer sich in seiner Farbe und/oder Farbtemperatur ändernden Beleuchtung zeigt, wobei die Beleuchtungsanordnung (1) vorzugsweise so angesteuert wird, dass bei einer Änderung einer Farbe und/oder einer Farbtemperatur der Beleuchtung eines auf der Anzeige (60) dargestellten Bildes (11) sich eine bei unmittelbarer Betrachtung des beleuchteten Bereichs (B) wahrgenommene Farbe und/oder Farbtemperatur der Beleuchtung nicht ändert und/oder mindestens ein anderes auf der Anzeige dargestelltes Bild (11, 12) den Bereich (B) mit einer Beleuchtung wiedergibt, deren Farbe und/oder Farbtemperatur sich nicht ändert.

8. Beleuchtungsanordnung (1) nach einem der vorangegangenen Ansprüche, wobei eine gewünschte Hauptbeleuchtungs-Farbe und/oder Farbtemperatur, mit der die Lichtquelle (2) den Bereich (B) in einem Normalbetrieb beleuchtet, anhand von auf einer Anzeige darstellbaren Bildern (11, 12) mit jeweils unterschiedlicher Farbe und/oder Farbtemperatur der Beleuchtung durch eine Bedienperson einstellbar ist.

9. Beleuchtungsanordnung (1) nach einem der vorangegangenen Ansprüche, wobei eine Auswertung der aufgenommenen Bilder (11, 12) in mindestens einem Betriebsmodus mittels der Steuerung (20) so erfolgt, dass Informationen aus mindestens zwei bei Beleuchtungen unterschiedlicher Farbe und/oder Farbtemperatur aufgenommenen Bildern (11, 12) in ein Ergebnisbild (14) integriert werden, wobei das Ergebnisbild (14) vorzugsweise auf einer Anzeige (60) dargestellt wird und/oder wobei die Auswertung der Bilder (11, 12) mittels einer Strukturerfassung (30) erfolgt.

10. Beleuchtungsanordnung (1) nach einem der vorangegangenen Ansprüche, wobei das separat ausgegebene und/oder ausgewertete Bild (11, 12, 13, 14) kontinuierlich aktualisiert wird und/oder zu durch die Steuerung (20) vorgegebenen Zeitpunkten aktualisiert wird und/oder auf Grundlage eines durch eine manuelle Eingabe einer Bedienperson verursachten Signals erstellt und/oder aktualisiert wird.

11. Beleuchtungsanordnung (1) nach einem der vorangegangenen Ansprüche, wobei es sich bei mindestens einer Kamera (10) um eine 2D-Kamera handelt, zur Aufnahme eines 2D-Bildes des beleuchteten Bereichs (B), und/oder wobei es sich bei mindestens einer Kamera (15) um eine 3D-Kamera handelt, zur Aufnahme eines 3D-Bildes des beleuchteten Bereichs (B), wobei vorzugsweise mindestens eine Kamera (10, 15) in mindestens einem aus VIS-Bereich, IR-Bereich und/oder UV-Bereich arbeitet.

12. Beleuchtungsanordnung (1) nach einem der vorangegangenen Ansprüche, wobei es sich bei der mindestens einen Lichtquelle (2) um mindestens eine Operationsleuchte (2) oder mindestens eine Endoskopie-Lichtquelle (2) handelt, wobei die Beleuchtungsanordnung (1) vorzugsweise mindestens einen Betriebsmodus aufweist, in welchem mittels einer Endoskopie-Kamera aufgenommene Bilder (11) eines Bereichs, der mittels der Endoskopie-Lichtquelle (2) beleuchtet ist, mittels der Steuerung (20) auf einer Anzeige (60) darstellbar sind, wobei vorzugsweise einzelne bei jeweils unterschiedlicher Farbe und/oder Farbtemperatur aufgenommene Bilder (11) der Endoskopie-Kamera mittels der Steuerung (20) in ein Ergebnisbild (14) ineinander integrierbar sind.

13. Verfahren zum Betreiben einer Beleuchtungsanordnung (1), insbesondere einer Beleuchtungsanordnung (1) nach einem der vorangegangenen Ansprüche, wobei die Beleuchtungsanordnung (1) mindestens eine Lichtquelle (2), mindestens eine Kamera (10) und eine Steuerung (20) umfasst, wobei das Verfahren ein Verändern der Farbe und/oder Farbtemperatur der Lichtquelle (2) umfasst und die Aufnahme von Bildern (11, 12) eines durch die Lichtquelle (2) beleuchteten Bereiches (B) mittels der Kamera (10) umfasst,
wobei das Verfahren folgende Schritte umfasst:
Ansteuern der Beleuchtungsanordnung (1) mittels der Steuerung (20), sodass die Lichtquelle (2) den beleuchteten Bereich (B) nacheinander mit Licht unterschiedlicher Farbe und/oder Farbtemperatur beleuchtet, und
separates Auswerten und/oder Ausgeben, insbesondere Darstellen, von mindestens einem ersten, bei einer ersten Farbe und/oder Farbtemperatur der Beleuchtung aufgenommenem Bild (11),
**dadurch gekennzeichnet,**
**dass** die Steuerung (20) die Lichtquelle (2) in mindestens einem Betriebsmodus so ansteuert, dass die Veränderung der Farbe und/oder Farbtemperatur so schnell erfolgt, dass dies für das menschliche Auge bei unmittelbarer Betrachtung des beleuchteten Bereichs (B) nicht wahrnehmbar ist.

14. Software mit Befehlen zur Durchführung eines Verfahrens nach Anspruch 13.

15. Software nach Anspruch 14 zur Integration von Informationen aus mindestens zwei bei Beleuchtungen unterschiedlicher Farbe und/oder Farbtemperatur aufgenommenen Bildern (11, 12) in ein Ergebnisbild (14).

## Claims

1. Lighting arrangement (1), which comprises at least one light source (2, 2'), with at least one camera (10, 15) and a control system (20), wherein the colour and/or colour temperature of the light source (2) is variable and the camera (10) captures images (11, 12) of a region (B) illuminated by the light source (2),
wherein the control system (20) controls the lighting arrangement (1) such that the light source (2) illuminates the illuminated region (B) successively with light of a different colour and/or colour temperature, wherein the control system (20) separately evaluates and/or outputs at least a first image (11) captured at a first colour and/or colour temperature of the lighting,
**characterized in that**
the control system (20) controls the light source (2) in at least one operating mode in such a way that the change in the colour and/or colour temperature takes place so quickly that this is not perceptible for the human eye when looking directly at the illuminated region (B).

2. Lighting arrangement (1) according to claim 1, wherein a main lighting colour and/or colour temperature, which is perceptible for the human eye and with which the light source (2) illuminates the region (B), can be set manually.

3. Lighting arrangement (1) according to any one of the preceding claims,
wherein the light source (2) is controlled in one operating mode such that the light source (2) illuminates the region (B) with a main lighting colour and/or colour temperature and switches from this main lighting colour and/or colour temperature for a period of time to the first colour and/or colour temperature, wherein during this period of time at least one image (11) is captured, which is evaluated and/or output separately, wherein the period of time is preferably so short that, on direct observation of the illuminated region (B), a perceived colour and/or colour temperature does not change due to the switch and is in particular less than 200 ms, preferably less than 50 ms.

4. Lighting arrangement (1) according to any one of the preceding claims, wherein the light source (2) is an LED light source (2), wherein the LEDs of the LED light source (2) are operated in a pulsed manner by means of the control system (20), wherein the control system (20) preferably controls the lighting arrangement (1) in at least one operating mode such that during the capturing of the image (11), the colour and/or colour temperature of the pulses does not change and/or that the pulses occurring during the capturing of an image yield a desired colour and/or colour temperature on average.

5. Lighting arrangement (1) according to any one of the preceding claims, wherein the control system (20) controls the lighting arrangement (1) in at least one operating mode such that illumination takes place successively with a plurality of different colours and/or colour temperatures, wherein the control system (20) separately evaluates and/or outputs several images captured in respectively different colours and/or colour temperatures of the lighting, wherein a switch preferably takes place in each case from a main lighting colour and/or colour temperature to one of the different colours and/or colour temperatures and back again.

6. Lighting arrangement (1) according to any one of the preceding claims, wherein in one operating mode, an image (11) is presented on a display (60) of the lighting arrangement (1), which image shows the illuminated region (B) in a colour and/or colour temperature of the lighting other than the colour and/or colour temperature of the light source (2), which is perceptible during the presentation of the image (11) when looking directly at the illuminated region (B).

7. Lighting arrangement (1) according to any one of the preceding claims, wherein in one operating mode, several images (11, 12) are presented on a display (60) of the lighting arrangement (1), which images show the illuminated region (B) in a respectively different colour and/or colour temperature of the lighting, and/or wherein in one operating mode at least one image (13) is presented on the display (60), which image shows the illuminated region (B) in illumination that is changing in its colour and/or colour temperature, wherein the lighting arrangement (1) is preferably controlled such that in the event of a change in a colour and/or a colour temperature of the lighting of an image (11) presented on the display (60), a colour and/or colour temperature of the lighting perceived when looking directly at the illuminated region (B) does not change and/or at least another image (11, 12) presented on the display reproduces the region (B) with illumination of which the colour and/or colour temperature does not change.

8. Lighting arrangement (1) according to any one of the preceding claims, wherein a desired main lighting colour and/or colour temperature with which the light source (2) illuminates the region (B) in normal operation can be set by an operator on the basis of images (11, 12) displayable on a display with a respectively different colour and/or colour temperature of the lighting.

9. Lighting arrangement (1) according to any one of the preceding claims, wherein an evaluation of the captured images (11, 12) in at least one operating mode takes place by means of the control system (20) such that information from at least two images (11, 12) captured in illuminations of a different colour and/or colour temperature is integrated into a resulting image (14), wherein the resulting image (14) is preferably presented on a display (60) and/or wherein the evaluation of the images (11, 12) is carried out by means of structure detection (30).

10. Lighting arrangement (1) according to any one of the preceding claims, wherein the separately output and/or evaluated image (11, 12, 13, 14) is updated continuously and/or is updated at points in time predetermined by the control system (20) and/or is produced and/or updated on the basis of a signal produced by a manual input of an operator.

11. Lighting arrangement (1) according to any one of the preceding claims, wherein at least one camera (10) is a 2D camera for capturing a 2D image of the illuminated region (B), and/or wherein at least one camera (15) is a 3D camera for capturing a 3D image of the illuminated region (B), wherein preferably at least one camera (10, 15) operates in at least one of the VIS spectrum, IR spectrum and/or UV spectrum.

12. Lighting arrangement (1) according to any one of the preceding claims, wherein the at least one light source (2) is at least one surgical lamp (2) or at least one endoscopy light source (2), wherein the lighting arrangement (1) has preferably at least one operating mode, in which images (11) captured by means of an endoscopy camera of a region illuminated by means of the endoscopy light source (2) can be presented on a display (60) by means of the control system (20), wherein preferably individual images (11) of the endoscopy camera captured in a respectively different colour and/or colour temperature can be integrated into one another by means of the control system (20) in a resulting image (14).

13. Method for operating a lighting arrangement (1), in particular a lighting arrangement (1) according to any one of the preceding claims, wherein the lighting arrangement (1) comprises at least one light source (2), at least one camera (10) and a control system (20), wherein the method comprises changing the colour and/or colour temperature of the light source (2) and comprises the capturing of images (11, 12) of a region (B) illuminated by the light source (2) by means of the camera (10), **characterised in that** the method comprises the following steps:
control of the lighting arrangement (1) by means of the control system (20) so that the light source (2) illuminates the illuminated region (B) successively with light of a different colour and/or colour temperature; and
separate evaluation and/or outputting, in particular presentation, of at least a first image (11) captured in a first colour and/or colour temperature of the lighting,
**characterized in that**
the control system (20) controls the light source (2) in at least one operating mode in such a way that the change in the colour and/or colour temperature takes place so quickly that this is not perceptible for the human eye when looking directly at the illuminated region (B).

14. Software, comprising commands for executing a method according to claim 13.

15. Software according to claim 14 for integrating information from at least two images (11, 12), captured in illuminations of different colour and/or colour temperature, into a resulting image (14).

## Revendications

1. Agencement d'éclairage (1), qui comprend au moins une source de lumière (2, 2'), avec au moins une caméra (10, 15) et une commande (20), la couleur et/ou la température de couleur de la source de lumière (2) étant modifiable et la caméra (10) enregistrant des images (11, 12) d'une zone (B) éclairée par la source de lumière (2),
la commande (20) commandant l'agencement d'éclairage (1) de telle sorte que la source de lumière (2) éclaire successivement la zone éclairée (B) avec une lumière de couleur et/ou de température de couleur différente, la commande (20) évaluant et/ou émettant séparément au moins une première image (11) enregistrée avec une première couleur et/ou température de couleur de l'éclairage,
**caractérisé en ce que**
la commande (20) commande la source de lumière (2) dans au moins un mode de fonctionnement de telle sorte que la modification de la couleur et/ou de la température de couleur s'effectue si rapidement que celle-ci n'est pas perceptible pour l'œil humain lors de l'observation directe de la zone éclairée (B).

2. Agencement d'éclairage (1) selon la revendication 1, dans lequel une couleur et/ou une température de couleur d'éclairage principale perceptible par l'œil humain, avec laquelle la source de lumière (2) éclaire la zone (B), peut être réglée manuellement.

3. Agencement d'éclairage (1) selon l'une quelconque des revendications précédentes, dans lequel la source de lumière (2) est commandée dans un mode de fonctionnement de telle sorte que la source de lumière (2) éclaire la zone (B) avec une couleur et/ou une température de couleur d'éclairage principale et passe de cette couleur et/ou température de couleur d'éclairage principale à la première couleur et/ou température de couleur pendant un intervalle de temps, au moins une image (11) étant enregistrée pendant cet intervalle de temps, laquelle est évaluée et/ou émise séparément, l'intervalle de temps étant de préférence suffisamment court pour que, lors de l'observation directe de la zone éclairée (B), une couleur et/ou une température de couleur perçue ne soit pas modifiée par le changement, et étant notamment inférieure à 200 ms, de préférence inférieure à 50 ms.

4. Agencement d'éclairage (1) selon l'une quelconque des revendications précédentes, dans lequel la source de lumière (2) consiste en une source de lumière LED (2), les LED de la source de lumière LED (2) fonctionnant par impulsions au moyen de la commande (20), la commande (20) commandant l'agencement d'éclairage (1) dans au moins un mode de fonctionnement de préférence de telle sorte que la couleur et/ou la température de couleur des impulsions ne changent pas pendant l'enregistrement de l'image (11) et/ou que les impulsions effectuées pendant l'enregistrement d'une image donnent en moyenne une couleur et/ou température de couleur souhaitée.

5. Agencement d'éclairage (1) selon l'une quelconque des revendications précédentes, dans lequel la commande (20) commande l'agencement d'éclairage (1) dans au moins un mode de fonctionnement de telle sorte que l'éclairage s'effectue successivement avec une pluralité de couleurs et/ou de températures de couleur différentes, la commande (20) évaluant et/ou émettant séparément plusieurs images enregistrées avec des couleurs et/ou des températures de couleur de l'éclairage respectivement différentes, en passant de préférence à chaque fois d'une couleur et/ou température de couleur d'éclairage principale à l'une des différentes couleurs et/ou températures de couleur et inversement.

6. Agencement d'éclairage (1) selon l'une quelconque des revendications précédentes, dans lequel, dans un mode de fonctionnement, une image (11) est représentée sur un affichage (60) de l'agencement d'éclairage (1), laquelle montre la zone éclairée (B) avec une couleur et/ou une température de couleur de l'éclairage différente de la couleur et/ou de la température de couleur de la source de lumière (2) qui est perceptible pendant l'affichage de l'image (11) lors de l'observation directe de la zone éclairée (B).

7. Agencement d'éclairage (1) selon l'une quelconque des revendications précédentes, dans lequel, dans un mode de fonctionnement, plusieurs images (11, 12) sont représentées sur un affichage (60) de l'agencement d'éclairage (1), lesquelles montrent la zone éclairée (B) avec une couleur et/ou une température de couleur de l'éclairage respectivement différente, et/ou dans lequel, dans un mode de fonctionnement, au moins une image (13) est représentée sur l'affichage (60), laquelle montre la zone éclairée (B) avec un éclairage dont la couleur et/ou la température de couleur varie, l'agencement d'éclairage (1) étant de préférence commandé de telle sorte qu'en cas de modification d'une couleur et/ou d'une température de couleur de l'éclairage d'une image (11) représentée sur l'affichage (60), une couleur et/ou une température de couleur de l'éclairage perçue lors de l'observation directe de la zone éclairée (B) ne change pas et/ou qu'au moins une autre image (11, 12) représentée sur l'affichage reproduise la zone (B) avec un éclairage dont la couleur et/ou la température de couleur ne change pas.

8. Agencement d'éclairage (1) selon l'une quelconque des revendications précédentes, dans lequel une couleur et/ou une température de couleur d'éclairage principale souhaitée, avec laquelle la source de lumière (2) éclaire la zone (B) dans un fonctionnement normal, peut être réglée par un opérateur à l'aide d'images (11, 12) pouvant être représentées sur un affichage avec chacune une couleur et/ou une température de couleur de l'éclairage différente.

9. Agencement d'éclairage (1) selon l'une quelconque des revendications précédentes, dans lequel une évaluation des images (11, 12) enregistrées s'effectue dans au moins un mode de fonctionnement au moyen de la commande (20) de telle sorte que des informations provenant d'au moins deux images (11, 12) enregistrées avec des éclairages de couleur et/ou de température de couleur différentes sont intégrées dans une image résultante (14), l'image résultante (14) étant de préférence représentée sur un affichage (60) et/ou l'évaluation des images (11, 12) s'effectuant au moyen d'une détection de structure (30).

10. Agencement d'éclairage (1) selon l'une quelconque des revendications précédentes, dans lequel l'image (11, 12, 13, 14) émise et/ou évaluée séparément est actualisée en continu et/ou est actualisée à des moments prédéfinis par la commande (20) et/ou est établie et/ou actualisée sur la base d'un signal provoqué par une entrée manuelle d'un opérateur.

11. Agencement d'éclairage (1) selon l'une quelconque des revendications précédentes, dans lequel au moins une caméra (10) consiste en une caméra 2D pour enregistrer une image 2D de la zone éclairée (B), et/ou dans lequel au moins une caméra (15) consiste en une caméra 3D pour enregistrer une image 3D de la zone éclairée (B), de préférence au moins une caméra (10, 15) fonctionnant dans au moins l'une des zones VIS, IR et/ou UV.

12. Agencement d'éclairage (1) selon l'une quelconque des revendications précédentes, dans lequel l'au moins une source de lumière (2) consiste en au moins une lampe d'opération (2) ou au moins une source de lumière endoscopique (2), l'agencement d'éclairage (1) présentant de préférence au moins un mode de fonctionnement dans lequel des images (11) enregistrées au moyen d'une caméra endoscopique d'une zone qui est éclairée au moyen de la source de lumière endoscopique (2) sont représentées sur un affichage (60) au moyen de la commande (20), des images individuelles (11) de la caméra endoscopique enregistrées avec une couleur et/ou une température de couleur respectivement différentes pouvant de préférence être intégrées les unes dans les autres au moyen de la commande (20) dans une image résultante (14).

13. Procédé pour faire fonctionner un agencement d'éclairage (1), notamment un agencement d'éclairage (1) selon l'une quelconque des revendications précédentes, l'agencement d'éclairage (1) comprenant au moins une source de lumière (2), au moins une caméra (10) et une commande (20), le procédé comprenant une modification de la couleur et/ou de la température de couleur de la source de lumière (2) et l'enregistrement d'images (11, 12) d'une zone (B) éclairée par la source de lumière (2) au moyen de la caméra (10),
le procédé comprenant les étapes suivantes :
la commande de l'agencement d'éclairage (1) au moyen de la commande (20), de telle sorte que la source de lumière (2) éclaire successivement la zone éclairée (B) avec une lumière de couleur et/ou de température de couleur différente, et
l'évaluation et/ou l'émission séparée, notamment la représentation, d'au moins une première image (11) enregistrée avec une première couleur et/ou température de couleur de l'éclairage,
**caractérisé en ce que**
la commande (20) commande la source de lumière (2) dans au moins un mode de fonctionnement de telle sorte que la modification de la couleur et/ou de la température de couleur s'effectue si rapidement que celle-ci n'est pas perceptible pour l'œil humain lors de l'observation directe de la zone éclairée (B).

14. Logiciel avec des instructions pour la mise en œuvre d'un procédé selon la revendication 13.

15. Logiciel selon la revendication 14 pour l'intégration d'informations provenant d'au moins deux images (11, 12) enregistrées avec des éclairages de couleur et/ou de température de couleur différentes dans une image résultante (14).
